# EUROPEAN PATENT APPLICATION

(11) **EP 1 604 914 A2**
(43) Date of publication of application: **14.12.2005**
(21) Application number: 05076759.9
(22) Date of filing: 09.10.2002
(51) Int. Cl.: B65D 81/00, B65D 5/50, B65D 5/48

(54) **Dunnage article**

(30) Priority: 01.11.2001 US 999091
(62) Divisional of application: 02802439.6
(71) Applicant: Johnson & Johnson Vision Care, Inc., Jacksonville, FL 32256 (US)
(72) Inventor: Duncan, Gregory Scott, Jacksonville, FL 32210 (US); Lepper, John M., Jacksonville, FL 32207 (US); Duis, Donnie J., Jacksonville, FL 32225 (US); Beaton, Stephen R., Jacksonville, FL 32250 (US)
(74) Representative: Mercer, Christopher Paul

(57) **Abstract**

A dunnage article for a container having a crosswise dimension and upwardly directed sidewalls, said dunnage article comprising a piece of unitary sheet material of elongated shape, said sheet material having a central portion (201) and at least two opposing end flaps (202A and 202B) that are delineated from said central portion (201) by first transverse fold lines (205A and 205B) said end flaps (202A and 202B) being proportioned to be bent along said first transverse fold lines (205A and 205B) and be seated against the inside of said upwardly directed sidewalls with a pressed fit when said dunnage article (200) is placed within said container, said central portion (201) having a length substantially equal to but less than the crosswise dimension of said container, said central portion having at least one opening therethrough sufficient to enable viewing of product located directly underneath said dunnage article (200) after said dunnage article is placed within said container.

## Description

### BACKGROUND OF THE INVENTION

### 1. Field of the Invention

The invention is directed to the automated packing and shipping of product, for example manufactured articles and goods, especially prescription products such as ophthalmic lenses. More specifically, the invention relates to a method and system for the packing and shipping of products where order information is used by a computer processing system in conjunction with various identifiers, preferably machine-readable identifiers, to automatically provide a container for the product, address, invoice, load and ship same in response to an order for that product. The invention further relates to a one-piece dunnage article universally useable in a container having one or more layers of product packed therein. The dunnage article further has specific openings therethrough which, once the dunnage article has been placed, allows a user to view and hence ascertain the type of product beneath it, without necessitating additional handling or removal of the dunnage.

### 2. Description of the Prior Art

Conventionally, product packing and shipping has been a labor-intensive endeavor. For example, in response to an order for a given product, a typical packing and shipping operation would require the manual assembly of a shipper box, retrieval of the ordered product from storage, packing of the shipper box with the product, and closing/sealing of the box. Additionally, dunnage in certain instances has to be placed into the shipper to reduce internal motion of the products susceptible to damage. Moreover, the shipper box must be properly addressed and contain the proper invoice; and it must also have the appropriate carrier designation thereon.

Heretofore, human operators were integrally involved throughout the packing and shipping protocol. In the particular realm of ophthalmic lenses, the packing and shipping process typically requires a human operator who, in sequence: selects and erects a shipper box; then checks the product cartons associated with the order by, for example, reading their barcodes to ensure that these completely and accurately fill the order. After manually loading these into the shipper box, an invoice is printed by the operator, who places it into the shipping box. Product information literature and other inserts, such as rebate coupons, as desired, are also hand placed into the box at this time. Other items and articles affiliated with the ophthalmic lenses, such as containers of saline solution, if called for by the order, are thereafter manually packed into the shipper, as is dunnage. The box is then closed by the operator and taped. A shipping label is thereafter printed out by the operator and applied to the box, which is then shipped to the customer who placed the order consistent with whatever carrier instructions obtain.

In terms of efficiency, a human operator going through the paces of the above-described packing and shipping process can complete, on average, about 40 orders, i.e. shipper boxes, per hour.

Industrially, output in this regard is increased by increasing the numbers of human workers involved in the process. As a consequence, however, direct labor costs are driven up; as is the amount of floor space needed for the additional staff to work in. Moreover, there is a certain amount of human error inevitably associated with the operation, which error tends to be multiplied by an increase in the number of workers -all of which lead to more inaccuracies in order fulfillment. Then, there are ergonomic injuries associated with the manual nature of the operation.

Accordingly, there is a need for an automated packing and shipping method and system which reduces the number of human operators and amount of floor space needed, with commensurate savings in costs, injuries and errors, all the while enabling an increase in output and accuracy.

Additionally, dunnage used to fill void space or otherwise protect products packed in a container conventionally comes in the form of plastic (e.g. polystyrene) particles of peanut or popcorn shape, or shredded paper. This loose form of dunage typically requires metering or other carefully controlled handling, the logistics of which becomes especially onerous when the containers each have different void spaces and hence need differing amounts of dunnage. The differences in void space in this regard can be due e.g. to the presence of different amounts or layers of products in one container versus another, depending upon the order or other circumstances. Moreover, once such conventional dunnage is in place, the user can no longer view the products packed in the container to ascertain their identity without further handling or removing the dunnage. Hence there exists a need for dunnage that does not need controlled metering, and that is universally useable across a spectrum of product fill layers and that need not be disturbed, once placed in the container, to view the products packed therein.

### SUMMARY OF THE INVENTION

The present invention satisfies the before-stated desiderata by providing a method and system for packing and shipping a product in an automated fashion. The inventive method comprises: i) providing a product in response to order information for a particular order, said product having a product identifier thereon; ii) providing a shipping container for said product; iii) providing at least one computer having access to one or more databases in which said order information and said product identifier are stored, said at least one computer causing the following steps to be performed automatically: (a) preparing a shipping label based on said order information, and attaching said shipping label to said shipping container, said shipping label being provided thereon with a label identifier that is associated with said particular order; (b) preparing an invoice for said product based on said order information, said invoice being provided with an invoice identifier thereon, said invoice identifier having correlation to said label identifier; (c) checking said invoice identifier against said label identifier to verify said correlation, and thereafter combining said invoice with said shipping container; and (d) checking said product identifier against said label identifier to verify said product is for said particular order, and thereafter packing said product into said shipping container based on said order information. The thus-packed container can then be shipped to whomever has placed the particular order in the first instance.

In another embodiment, the invention pertains to a system for the automated packing of product, the system comprising (a) a computer having a database for storing i) order information on a particular order for a product, and ii) product identifier information, said product identifier being located on said product; (b) means for providing at least one shipping container for products associated with said particular order; (c) means for preparing a shipping label for said product based on said order information, said shipping label having thereon a label identifier that is associated with said particular order; (d) means for attaching said shipping label to said shipping container; (e) means for preparing an invoice for said particular order based on said order information, said invoice having thereon an invoice identifier that correlates to said label identifier; (f) means for comparing said invoice identifier to said label identifier to verify that said invoice is for said particular order; (g) means for inserting said invoice into said shipping container; (h) means for comparing said product identifier on said product to said label identifier to verify that said product is for said particular order; and (i) means for packing said product into said shipping container. The system also contemplates means for shipping the packed container, including means to ship by designated carrier.

In another aspect, the present invention is directed to a dunnage article for a container having a crosswise dimension and upwardly directed sidewalls, said dunnage article comprising a piece of unitary sheet material of elongated shape, said sheet material having a central portion and two opposing end flaps that are delineated from said central portion by first transverse fold lines, said end flaps being proportioned to be bent along said first transverse fold lines and be seated against the inside of said upwardly directed sidewalls with a pressed fit when said dunnage article is placed within said container, said central portion having a length substantially equal to but less than the crosswise dimension of said container, said central portion having at least one opening therethrough sufficient to enable viewing of product located directly underneath said dunnage article after said dunnage article is placed within said container

### BRIEF DESCRIPTION OF THE DRAWINGS

FIGURE 1 shows a preferred layout (top view) for automated packing and shipping pursuant to the invention.
FIGURE 2 shows a preferred embodiment of the dunnage article contemplated by the present invention.
FIGURES 3A and 3B depict the preferred dunnage article of FIGURE 2 situated in a container having one and two levels of product respectively; FIGURE 3C depicts the preferred dunnage article of FIGURE 2 in a folded position.

### DETAILED DESCRIPTION OF THE INVENTION

For purposes of the present application: the term "product" whether singular or plural refers to materials (raw or otherwise), goods and/or articles of manufacture. Articles of manufacture include, without limitation, prescription products, including for example ophthalmic lenses such as hard, soft, rigid and gas permeable contact lenses, intra-ocular lenses and lenses for eyeglasses.

The term "order information" refers to information concerning an order or request placed by a user or customer such as a doctor, patient or distributor and includes without limitation types of products requested, quantity of each, pricing of each, shipping address, designation of carrier to be used, e.g. U. S. Postal Service, private carrier or the like, and any other information typically associated with the provision and delivery of a product in response to an order. Order information can also comprise instructions to include with a particular order various pre-printed inserts, such as product literature, customer literature (including e.g. personalized literature such as thank you or reminder cards), rebate coupons and the like. Additionally, for ophthalmic lenses, soft contact lenses in particular, order information can further comprise instructions to include as part of a particular order, articles affiliated with such lenses, including without limitation containers of saline or other solutions typically used in the wearing, care and maintenance of contact lenses.

The term "identifier" refers to a number, barcode, multi-dimensional matrix such as a two-dimensional matrix, inductive transmitting device, radio frequency chip, or any of the like. It is preferred that each identifier contain some unique information about each product or item having one. The identifier may be machine readable and/or human readable. A machine-readable identifier is preferred, the machine readable identifier preferably having information stored in a database associated with the identifier. For packaging in this regard, the only way to know which product is in the package is to access the information in the database by inputting the identifier in to the database, via a bar code or other optical reader or the like. The preferred identifier is in the form of a bar code or multi-dimensional matrix such as a two-dimensional matrix.

The invention will now be further described in the terms its preferred embodiment where the product is a prescription product, more preferably an ophthalmic lens. After order information as hereinbefore described is generated in response to an order or request for a product placed, e.g. by a doctor, distributor or patient seeking a refill, packages containing the requested product are collected and sent to the packing and shipping station of the invention. Order information in this regard is generated and controlled by at least one computer having access to one or more databases in which the relevant information concerning the order is stored, updated and otherwise manipulated. A product package, especially in regard to ophthalmic lenses, is preferably a carton, but other packaging such as a bag, plastic wrap, envelope, pouch, can, box, bottle, tray and the like are contemplated. In the case of ophthalmic lenses, such packaging is referred to as secondary packaging, the primary being a blister pack or the like. Typically, each package has product identifier thereon as heretofore described. Product is collected to fill the particular order by, for example pulling same from storage or by providing same directly from the manufacturing line.

While conventional techniques for assembling product in fulfillment of a particular order can be employed in the present invention, a preferred practice for product handling and assemblage is described in "APPARATUS AND METHOD FOR AUTOMATED WAREHOUSING AND FOR FILLING ORDERS FROM MULTILAYER INVENTORIES" (VTN-0453), U. S. Serial No.09/494,860, filed February 1, 2000, the entire contents of which are hereby incorporated by reference. For example, and without limitation to the present invention, a multiplicity of customer orders for the same or different products are received by telephone, mail, internet, facsimile or any other method, and are organized into one or more databases (not shown). Products are picked from inventory to fulfill the various orders, the sequence of such picking can be order-by-order or can be based on priorities of level of demand for a given product, convenience of storage location and the like. One or more robotic arms or similar devices can be utilized to pick products from inventory consistent with the foregoing protocols. Products thus collected can be sorted into their respective orders by known techniques, or in a preferred practice embodiment, can be automatically sorted in the manner described in "ORDER BUILDER" (TPC 48-00), U. S. Serial No.09/903,249, filed July 11, 2001, the entire contents of which are hereby incorporated by reference.

### Data Tracking:

The packing operation of the present invention is preferably controlled by at least one computer having access to one or more databases in which are stored at least i) order information concerning a particular order, and 2) information concerning the product identifier.

In a preferred practice, a host computer having access to the databases aforesaid operates in conjunction with one or more PLCs which are locally situate the packing system of the invention and control some or all of the steps hereinafter described. Data available to the PLCs from the host in this regard through e.g. an ethernet connection includes without limitation: identifier information (e.g. barcode information), shipping container configuration information e.g. information which matches the shipping container to: the label identifier, the product identifier (located for example on the product cartons constituting the order), the insert identifier (for any inserts required), the invoice identifier, dunnage required, articles (such as containers of saline solution that are required), and any priority tape needed for a specific carrier designated by a particular order. Also available to the PLCs is information as to whether an order is partial (e.g. not all product cartons constituting a particular order were accounted for by the packing system as having been loaded into the requisite shipping container) and hence is to be rejected as part of the packing operation of the invention; and information as to the status of a completed shipping container.

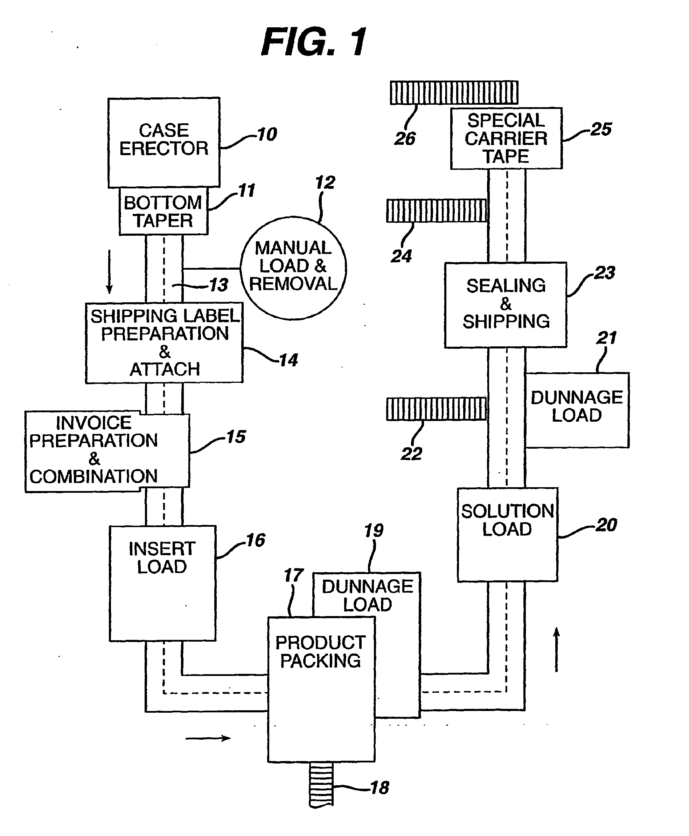

Logistically, and as more specifically elaborated upon in the preferred embodiment of Figure 1 described below, the packing operation of the present invention transfers a shipping container provided for a particular order from one station, as described herein, to the next while also transferring the associated data with the shipping container. The shipping container is transported through the packing system by transport means known in the art, preferably by way of one or more conveyor belts and the like. In general, the invention contemplates the provision of a shipping container or containers suitable for packing the components of a particular order, the shipping container either being selected and provided manually, or selected and provided automatically based upon the order information. In a preferred practice, the shipping container is erected and bottom taped at a case erector (or optionally the container is provided to the packing system fully erected in the first instance) and transported by a conveyor belt to a shipping label preparation and attachment station whereat the shipping label is prepared and applied to the shipping container. The shipping label has thereon necessary information regarding address, carrier and the like and includes a label identifier, e.g. a barcode, that matches up and to and is associated with the particular order (e.g. it contains the order number that has been assigned to the order) to be packed. The label is thereafter attached to the shipping container and the label identifier is preferably then read by a scanner (e.g. barcode reader) to ensure the label identifier is readable. The information available from the label identifier is preferably used in conjunction with, for example a shift register, to track the shipping container as it proceeds throughout the packing system of the invention. The shipping container is then conveyed to an invoice preparation and loading station whereat the invoice for the particular order being packed is prepared; the invoice further comprises an invoice identifier (e.g. a barcode) which correlates to the label identifier (e.g. both contain the order number for the particular order being packed). Preferably, the invoice identifier is scanned as is the label identifier to verify within the databases by means known in the art, that the invoice is correctly matched to the shipping container presently at the invoice preparation station. Upon verification, the invoice is placed into the shipping container, and in one embodiment can be folded to accommodate same. This shipping container is then conveyed to one or more insert loading stations whereat product and/or customer literature is optionally placed into the shipping container. In practice, if e.g. a particular order requires certain literature accompany it, the subject insert can have thereon an insert identifier (e.g. a barcode) which is scanned along with the label identifier on the shipping container to verify that the insert belongs to the order associated with that container, after which the insert is loaded. Sensors can be used to ensure deposit has occurred. Separately, products, typically in cartons having product identifiers thereon (e.g. barcodes corresponding to product type and the like) enter the packing system of the invention at the product packing station. There, the product identifiers on the cartons are scanned as is the label identifier on the shipping container then located at the product packing station, to verify as aforesaid that the cartons are correctly matched up with the shipping container and that both are for the same order. Once verified, the cartons are packed into the shipping container using techniques known in the art whereafter the packed container is optionally conveyed to a dunnage load station and/or further optionally an article insert station (articles in this regard include items such as containers of saline solution that may be required by a particular order). Vacuum detection is used at these stations to ensure that dunnage and/or solution has been loaded into the shipping container; vacuum detection can also be used to confirm that product has actually been loaded into the shipping container and can similarly be used to confirm that shipping labels have been applied to the shipping container. The shipping container is then transported to top taping station whereat it is sealed and then to out-feed conveyers for whichever carrier has been designated by the order information or otherwise, e.g. if U.S. Postal Service (USPS) the shipping container can proceed to a priority tape station whereat the appropriate USPS tape is applied e.g. to three sides of the container. One or more reject lanes are also contemplated; for example, in a preferred mode one reject lane is designated for partial orders (e.g. shipping containers that the system has determined does not contain all the components associated with the order); in a preferred practice, the partial order rejects are cured by completion of the order and then shipped. Another reject lane is for shipping containers that have had errors other than partial orders as identified by the system of the invention. In the practice of the invention about 900 orders/hour (on average) can be packed and shipped.

The invention will now be further described with reference to the preferred embodiment depicted in Figure 1. Those in the art will appreciate variations from that which is herein described, all of which variations are within the scope and spirit of the present invention.

### Shipping Container:

Shipping containers contemplated for use in the present invention are those known in the art. In practice, the shipping container is provided to the packing system of the invention having been pre-selected and optionally erected and taped as necessary. In another embodiment, the shipping container or containers is selected as part of the present invention based on the order information for a particular order, e.g. based on the size and the number of product packages required to fill the order; for example, in the databases tapped by the computer may be a table that cross references container size to number and/or type of products or other parameter that could drive the selection. Without limitation, shipping containers contemplated by the invention, also referred to herein as shippers or boxes, include containers made of corrugate, paperboard, thermoformed plastics, erected containers and foamed containers; and including rectangular containers, elongated containers, drums, crates and molded products such as e.g. molded trays. The preferred shipping container is a Regular Slotted (or Standard) Container (RSC), the types and sizes of which include those known in the art.

In one embodiment, the shippers are erected in-line, i.e. at the shipping and packing station. In a preferred practice, flat RSC shippers are provided. These are preferably loaded into one or more hoppers (not shown) which feed one or more case erectors 10 referring to Figure 1. In situations where different styles and sizes of shippers are in use, multiple hoppers can be employed. While preferred, hoppers need not be utilized, and the shippers can be manually erected, or purchased partly or fully erected in the first instance, and introduced into the packing system of the invention. The case erector removes the boxes from the hopper, forms the box, folds the bottom flaps, and comprises a bottom taper 11 which tapes the bottom flaps to seal that end of the shipper; the case erector 10 then dispenses the erected, bottom taped shipper downstream to the next step in the packing process of the invention. A suitable case erector and bottom taper in this regard is the commercially available "Combi 2-EZ" from Combi Packaging Systems. In a particular practice of the invention, the case erector has container stock manually loaded therein, with automatic erecting/taping following thereafter.

In another embodiment, the shippers can be erected off-line. This may be desirable when, under given circumstances, the number of shippers required have sizes and/or styles so disparate or numerous that use of a hopper is rendered impractical; conversely, where quantities of a given shipper style or size is too low to warrant hopper loading in the first instance, it may be more expedient to erect these elsewhere, off-line. Other situations where the shipper may be erected off-line include those where it is of unusual configuration and does not fit into an available hopper. When erected off-line it is preferred to introduce the erected shippers into the packing system of the invention by way of a Manual Load Station 12. In another preferred embodiment the Manual Load Station 12 also has facilities to remove shippers erected by case erector 10 if, for example, the shippers have been damaged or are defective; or they can be removed for any other purpose at this point.

In the practice depicted in FIGURE 1, the erected shippers, whether erected by case erector 10 or off-line and introduced at manual load station 12, are placed on transport means 13 which comprise any such means known in the art including without limitation, a conveyor, e.g. belt, chain, mat and the like. In a preferred mode, the conveyor is a modular conveyor and is employed throughout the packing system of the present invention, an example of which is the Series 300 Timing Belt Profiled Conveyor commercially available from Q.C. Industries, Inc.

### Shipping Label Preparation & Attachment:

All steps described herein are automatically performed: With reference to the preferred embodiment depicted at FIGURE 1, an erected shipping container slated for a particular order proceeds along conveyor 13 to the Shipping Label Preparation and Attachment Station 14 whereat a shipping label containing, e.g. the address to which the order is to be shipped, carrier designation, e.g. U. S. Postal Service, private carrier and the like, and any other shipping instructions such as expedited or special delivery instructions, is prepared. The label is prepared based on the order information stored in the database aforesaid. In the practice of the invention, the label already has or in a preferred practice is prepared with the shipping instructions to have a label identifier thereon. The label identifier is associated with the particular order for which the label is being prepared. In one embodiment, the label identifier is machine-readable, preferably a barcode or the like, which for example can signify the order number assigned to the particular order. This can be printed on the label as part of its preparation, or can be separately prepared and thereafter attached to the label. The label may be prepared by methods known in the art, including any printing method, e.g. thermal, laser, electrophotographic, ink jet and pad printing. The label identifier is used to track and coordinate the container to which the label is attached, with subsequent steps of the inventive method as described herein.

Once prepared, the label is attached to the shipping container that has been selected for that same order. In circumstances where more than one container is needed to pack and ship a particular order, a commensurate number of labels can be prepared for attachment with each of the same. In another embodiment, a single master label can be prepared and attached to the collection of containers that are in turn bounded together to complete the order. The label may be attached to the container by any means known in the art, including without limitation, pasting, clipping, stapling and the like. In one embodiment, the label itself has an adhesive on one side of it; in another embodiment, the label is prepared and slipped into an envelope, pouch or similar holder of sufficient transparency such that the address and carrier information can be seen therethrough. Attachment of the shipping label and the container is performed automatically. For example, in one practice the label is printed to have carrier and address information and a barcode signifying the order number, and is then applied directly to the container; label preparation and application equipment in this regard is without limitation commercially available from Label-Aire. In a preferred practice of the invention, a shipping label is prepared and applied to a shipper in a time frame of up to about every 10 seconds; more preferably between about every 2 to about every 4 seconds.

In a preferred embodiment, the shipping label is attached to the shipping container such that the section of the label whereon the label identifier appears wraps around the side of the container effective to facilitate reading of the identifier, hence tracking of the container, by scanners or sensors, e.g. barcode readers, as the container proceeds on the conveyor through other areas of the inventive packing and shipping system.

### Invoice Preparation & Combination:

All steps described herein are automatically performed: With reference to the preferred embodiment depicted at FIGURE 1, the shipping container now having attached thereto the shipping label with the label identifier, proceeds along conveyor 13 to the Invoice Preparation and Combination Station 15 whereat an invoice is prepared based on the order information. The invoice can include, without limitation, conventional information such as the quantity, value or prices and charges for the products being shipped as well as an itemized statement of account for same. In the practice of the invention, the invoice either already has or is further prepared to have an invoice identifier thereon that correlates to the label identifier and hence the particular order for which the invoice is being prepared. This invoice identifier can be the same as the label identifier, or it can be different as long as it can be correlated with the label identifier thus enabling correct match up of the invoice to the shipping container for which it is for. Correlation in this regard can be automatically performed by the host computer, or by more locally situated computer such as shop floor controllers and the like. In one embodiment, the invoice identifier is a barcode, preferably the same as that utilized as the label identifier; in a still more preferred practice, both signify order number for the particular order to which they relate. The invoice can be prepared by any methods known in the art including any printing method, e.g. thermal, laser, electrophotographic, inkjet and pad printing.

In the practice of the invention, both the invoice identifier and the label identifier on the shipping container are read by the same or different scanners or other sensor means to input same into the one or more databases as hereinbefore described whereby the computer or computers associated with said databases and the packing operation then compare the invoice identifier to the label identifier to verify that the invoice and the shipping container are correctly matched up. The comparison can utilize techniques known in the art; for example, the datapoints (which, for example, can be alpha numerical values) against which the identifiers are checked can be the same (e.g. the barcodes can be the same); or they can be different (e.g. the barcodes can be different and are correlated to some other value or values to establish a match). This comparison ensures that the invoice is matched to the correct shipping container before it is combined with same. In a preferred practice, if verification is not received, the order is denominated as rejected. In one embodiment, the invention can be operated to permit a predetermined number of consecutive rejections without stopping or otherwise shutting down the automated pack out system; in such cases, it is preferred that rejected orders are shunted to reject lane 22 whereat corrective action can be taken by operators to rework the order to completion. The system can also be operated to shut down after a given number of orders have been rejected whereupon operators can redress the situation causing the stoppage.

The invoice is combined with the container by any means known in the art including attaching same to the outside by pasting or the like; or placing same inside the container. In a preferred practice, the invoice is inserted into the container after the invoice identifier has been compared and matched with the label identifier. The invoice in this regard may be placed directly into the container as is; or it may be folded to accommodate the internal dimensions of the shipper. The invoice identifier must be read either before the invoice is folded, or the folding must be such that the identifier remains readable afterward.

In a preferred embodiment, the invoice is printed from order information received from the computer storing same. An invoice identifier in the form of a barcode signifying order number is also printed on the invoice. The invoice is then folded so that it fits into the shipping container, e.g. it is folded in half, and then folded again 90 degrees in half; the folding is such that the barcode is still machine readable prior to placement into the container. Equipment for invoice preparation in this regard is without limitation commercially available from Peripheral Systems (PSI). The invoice barcode is then read and compared by the host system to the barcode on the label either by accessing the label barcode from databases or by reading the label barcode again; if the label barcode matches, the invoice is placed into the shipper. In a preferred practice of the invention, an invoice is printed, folded, verified and inserted into its shipper in a time frame of up to about every 10 seconds; more preferably between about every 2 to about every 4 seconds.

### Insert Loading:

In an optional practice of the present invention, as depicted in the preferred embodiment of FIGURE 1, various inserts may be automatically placed into the shipping container at the Insert Load Station 16. These inserts can include pre-printed product literature and customer literature, such as rebate coupons; and can include personalized customer information such as thank you cards or reminder cards indicating as a convenience when the next refill should be ordered. Inserts may be placed as a matter of course into every shipping container as it proceeds along conveyor 13 irrespective of the product; or certain inserts may constitute part of the order information, e.g. the host or local computer may require specific literature accompany a particular product or a particular order. More than one inserter can be present with the various inserts stacked in one or more buffers which feed the inserters. For example, a first inserter can be loaded with inserts that are to be placed into every shipper, a second inserter can be loaded with inserts that are peculiar to a certain product and so on.

In one embodiment, where a particular insert is required for a particular product based on the order information, the insert has an insert identifier thereon, e.g. a barcode or similar identifier consistent with those delineated above. In one practice of this embodiment, both the label identifier on the shipping container and the insert identifier on the insert are read by the same or different scanners or other sensor means to input same into the one or more databases as hereinbefore described whereby the computer or computers associated with said databases and the packing system of the invention compare the label identifier to the insert identifier to verify that the insert and the shipping container are correctly matched up. In another practice of this embodiment, the Insert Loading Station 16 can be integral with the Invoice Preparation and Combination Station 15. In this practice, the label identifier information has already been inputted as part of the Invoice Preparation and Combination Station 15 and local tracking of the label identifier for purposes of the Insert Loading Station 16 has already been accomplished; hence only the insert identifier need be read for the comparison aforesaid to take place. As appreciated by those in the art, the Insert Loading Station 16 and the Invoice Preparation and Combination Station 15 can be reversed in sequence, the inputting of identifier information being adjusted accordingly.

Preferably, a sensor, such as without limitation a retro-reflective photoelectric (light beam) sensor, may be employed in conjunction with the inserters to ensure that a given insert has been loaded into the container. In a commercially available inserter employable in the present invention is the Longford 350 series of high speed feeders which can be obtained with detectors to verify placement of the insert.

### Product Packing:

Product needed to fill a particular order is collected in response to the order information and is provided to the packing and shipping system of the instant invention by way of e.g. the Product Packing Station 17 of the preferred embodiment depicted at FIGURE 1. In situations wherein the product is an ophthalmic lens, such as a soft contact lens, the product is in a product package, such as a carton, as hereinbefore described, the product package having a product identifier thereon. Multiple packages of the same or different products may be required to completely fill a particular order. As described above, the packages associated with a particular order can be collected by means known in the art; or alternatively in a preferred practice, they may be sorted in the manner described in "ORDER BUILDER" (VTN-546), U. S. Serial No.09/903,249, filed July 11, 2001, the entire contents of which are hereby incorporated by reference, and sent directly to the automated packing system of the present invention. Optionally, as described in the "ORDER BUILDER" aforesaid, the packages can be sorted and then sent to a label application station (not shown) whereat labels, preferably labels that have been customized in accordance with customer instructions, are applied to the packages. In a preferred practice, labels and/or packages are customized as described in "CUSTOMIZED PRESCRIPTION PRODUCT PACKAGING AND METHOD AND SYSTEM FOR PRODUCING CUSTOMIZED PRESCRIPTION PRODUCT PACKAGING" (VTN-0458), U. S. Serial No.09/494,859, filed February 1, 2000, the entire contents of which are incorporated herein by reference. After label application in this regard, the packages can be sent to the automated packing system of the invention. Packages can be conveyed to the system of the present invention by means known in the art, including e.g. by conveyor means shown in FIGURE 1 as 18.

At Product Packing Station 17 as shown in FIGURE 1, product packages arrive from conveyor 18. In a preferred practice, the products associated with a particular order arrive together before the next order in the sequence, any sorting in this regard having already been performed as described above. In one practice of the invention, the shipping container or containers designated for the same order arrives at Product Packing Station 17 contemporaneously with said packages; alternatively, the shipping container or containers can arrive at Packing Station 17 before the products to await same; or after the products. The product identifiers on the packages, and the label identifier on the shipping container are read by the same or different scanners or other sensor means to input same into the one or more databases as hereinbefore described whereby the computer or computers associated with said databases and the packing operation compare the product identifiers and the label identifiers to verify that products are matched up to the correct shipping container and that the correct number of cartons associated with the particular order are available before packing begins. Mechanically, packing can employ devices known in the art such as without limitation a pneumatic pick-and-place unit having e.g. vacuum grippers.

In a preferred practice, products will be packed into the shipping container until the order is fulfilled or until the container is full. As appreciated by those in the art, more than one shipping container may be required to completely pack a particular order; in a preferred practice of the invention, if more than one shipping container is needed to pack out a particular order, they will be filled seriatim at Product Packing Station 17 until the entire order has been loaded into same. In a preferred mode especially suited for ophthalmic lenses, if there are 8 product cartons or less associated with a particular order, the shipping container will be packed and then proceed to the next station as described infra., e.g. the Dunnage Station; if there are more than 8 cartons associated with a particular order, the first set of 8 will be placed in the shipping container whereafter the next set of 8 cartons will be placed on top of the first set in the same shipping container, the shipping container (now containing 16 product cartons) then proceeding to the next station infra. As those in the art will appreciate, variations of this packing protocol exist and all are contemplated by the present invention. In a preferred embodiment, the load size for a shipping container will be a minimum 1 product carton with an average load size of 8 product cartons. And while the cycle time for the method of the invention can be based upon any of the steps comprising same, it is preferred if the system cycle time is based upon the product packing step; a preferred cycle time in this regard is 4 seconds per shipping container for a single layer carton load, and 8 seconds per shipping container for a double layer carton load.

### Dunnage:

In another aspect, the present invention contemplates a one-piece dunnage article that is universally useable in containers having one or more layers of product, including e.g. ophthalmic lens products in cartons, packed therein. The dunnage article of the present invention is formed of a unitary sheet of material, e.g. stock material, including without limitation paperboard of one or more plies, corrugated board (which is preferred) and plastic. While the dunnage of the invention can be employed in a variety of containers, those having a crosswise dimension and upwardly directed walls, which walls can be substantially normal to the plane of the bottom of the container but need not be, i.e. the walls can be outside the plane perpendicular to the bottom. Suitable containers include, without limitation, those denominated as Regular Slotted (or Standard) Containers (RSCs), which containers have a length and width and substantially vertical sidewalls (e.g. the upwardly directed sidewalls that are substantially normal to the plane of the bottom of the container). In practice, containers contemplated by the invention may, when packed, have one, two or more layers of product cartons therein. The unitary dunnage article of the invention is configured to seat against the inside of the upwardly directed sidewalls with a pressed fit, irrespective of the number of layers of product packed in the container, thereby preventing damaging movement and shifting from occurring. In addition, the dunnage article of the invention improves the structural stability of the container in which it is used, making same more robust.

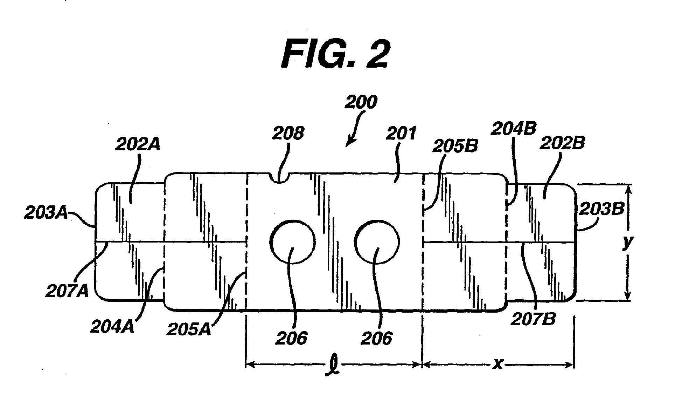

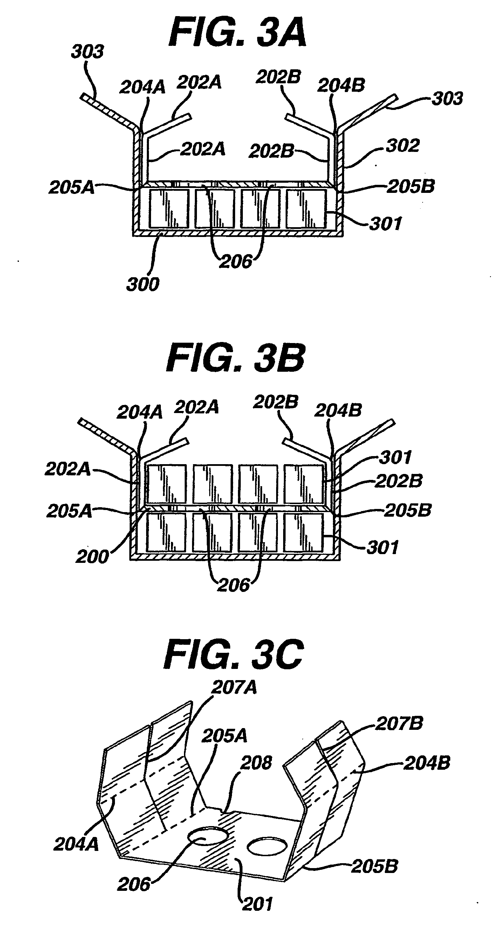

FIGURE 2 depicts a preferred embodiment of the dunnage article of the invention. Said article 200 is of generally eleongated shape, the preferred shape in FIGURE 2 being generally rectangular. The unitary sheet material comprising the dunnage article is divided into three sections: a central portion 201 and at least two opposing end flaps 202A and 202B which are delineated from central portion 201 by first transverse fold lines 205A and 205B. More particularly, end flap 202A is defined by first transverse fold line 205A and outer edge 203A; similarly end flap 202B is defined by first transverse fold line 205B and outer edge 203B. Transverse fold lines for purposes of the invention are formed in ways know in the art, such as by scoring or other weakening, so that the dunnage article will predictably bend along same as shown, for example, in FIGURE 3C.

End flaps 202A and 202B are proportioned to be bent along the first transverse fold lines 205A and 205B when dunnage article 200 is placed within a container such that the flaps will be bent and seated against the upwardly directed walls with a pressed fit. In conjunction with this, the central portion 201 is configured to have a length "1" that is substantially equal to but less than the crosswise dimension of the subject container. The central portion is thus preferably designed to just fit into the container thereby covering the entirety of the product cartons packed thereunder. Central portion 201 further has at least one opening 206 therethrough (two openings are preferred as shown in FIGURE 2). The openings can be of any shape; circular is preferred. Openings 206 are sized sufficient to enable viewing of packed product, e.g. product identifier information, on cartons located directly underneath dunnage article 200 after it is placed in the subject container. This allows a user to ascertain what the products are without having to handle or remove the dunnage from the coantiner.

In a preferred aspect, central portion 201 has an alignment notch 208 thereon; notch 208 is useful, for example, in helping to properly situate and load the dunnage articles in an automated system. One or more notches are contemplated by this term; preferably, one alignment notch 208 is present and is offset from the center of central portion 201. Alignment notch 208 allows one to determine proper direction and placement of the dunnage, the notch being further preferably offset as illustrated in FIGURE 2 to allow determination of which side of the dunnage is face up or face down. In the preferred design shown in FIGURE 2, opposing end flaps 202A and 202B each have respective mid longitudinal slits 207A and 207B therethrough, said slits preferably extending from outer edges 203A and 203B to first transverse fold lines 205A and 205B. Dunnage article 200 further preferably has second transverse fold lines 204A and 204B that are substantially parallel to said first transverse fold lines 205A and 205B, more preferably located between outer edges 203A and 203B and first transverse fold lines 205A and 205B. Slits 207A and 207B and second transverse fold lines 205A and 205B independently facilitate placement and fitting of dunnage article 200 into a subject container.

For purposes of convenience, the dunnage article depicted at FIGURE 2 and as described herein is referred to as "U-board" dunnage.

FIGURES 3A and 3B depict dunnage article 200 in a container such as a rectangular slotted container 300. In FIGURE 3A, container 300 is packed with one layer of product cartons 301. Dunnage article 200 is shown with end flaps 202A and 202B bent at first transverse fold lines 205A and 205B into a press fit against upwardly directed sidewalls 302. Central portion 201 covers substantially all of product cartons 301 with openings 206 sized sufficient to permit viewing of product, e.g. viewing of product identifier information printed on the carton. Second transverse fold lines 204A and 204B are also shown, the end flaps shown as being further bent to accommodate closing of the top 303 of container 300. In FIGURE 3B, two layers of product 301 are shown packed into container 300. In this embodiment, dunnage article 200 of the invention is situated between the first and second layers of product 301. End flaps 202A and 202B are bent along first transverse fold lines 205A and 205B respectively. The flaps are further bent along second transverse fold lines 204A and 204B and across the top layer of product or a portion thereof. Second transverse fold lines 204A and 204B are can but need not be not placed equidistant from their respective outer edges, 203A and 203B. Thus in one embodiment, second transverse fold line 204A is closer to outer edge 203A than second transverse fold line 204B is to edge 203B. This differential allows accommodation of differently sized product cartons. In another preferred embodiment, the end flaps of dunnage article 200 have one or more slits 207A and 207B; in instances where less than a full layer of product cartons are packed into the container, the dunnage article splits at the slits sufficient to secure the uneven number of cartons. By way of example, where a shipping container can accommodate up to 16 ophthalmic lens cartons (8 to a layer) but less than that are packed, e.g. 14 cartons are packed: the bottom layer has 8 cartons and the top layer has 6 cartons leaving an uneven number of cartons on one side of the top layer, the end flaps will split at the slit to help secure the uneven number of cartons.

FIGURE 3C depicts the preferred dunnage article of the invention described above in a partly folded state.

In a particularly preferred embodiment, referring to FIGURE 2, the length "x" of each opposing end flap 202A and 202B is approximately one half the length "1" of central portion 201. For example, given an RSC having a length of about 6 and 3/8 inches, a width of about 6 inches and a height of about 3 and ¼ inches, a suitably sized dunnage article contemplated by the invention has a central portion length ''1'' of about 6 ¼ inches; the two opposing end flaps 202A and 202B each have a length "x" of about 3 5/8 inches, and a width "y" of about 5 ¼ to about 5 ½ inches; two circular openings 206 are present, each have a diameter of about 2 inches.

### Dunnage Load:

In an optional embodiment, dunnage may be added to the shipping container either before or, preferably, after product is packed therein, which configuration is depicted at Figure 1 where Dunnage Load Station 19 is located downstream of Product Packing Station 17. Dunnage may be of the types known in the art including without limitation peanut- or popcorn-shaped particles of plastic or other suitable material of construction, shredded paper and the like which can be introduced into the shipping container in metered amounts. A preferred dunnage particularly suited to the practice of the present invention is U-board dunnage as depicted in Figure 2 and described herein. U-board dunnage contemplated by the invention has the advantage of fitting the shipping container irrespective of whether it contains one layer of product cartons or two. Thus the same dunnage can be used in all cases without requiring metering and the like. In a preferred embodiment, a hopper (not shown) contains a stack of flat U-board pieces. Placement of the U-board is achieved by means known in the art, e.g. by use one or more grippers having a vacuum actuated placement head or the like, which obtains the flat U-board from the hopper and deposits it into the shipping container effective to dampen movement of the product cartons. Placement is preferably controlled by the computer or computers having access to the one or more databases aforesaid. For example, if a shipping container has one layer of cartons, as ascertainable from said one or more databases and the number of cartons associated with a particular order, the U-board is preferably placed about half way down the container; if the shipping container has two layers of product cartons, it is preferred if the U-board is placed between the first and second layer. Placement can be checked by techniques known in the art, including e.g. by monitoring the vacuum on the placement head. In yet another preferred practice, discussed below, if a particular order requires the inclusion of articles into the shipping container, for example, a particular order may require packing of one or more containers of saline solution, then dunnage may be dispensed with, the saline or other solution container effectively serving the purpose of preventing unwanted motion of product during shipping and handling.

In yet another optional practice, as depicted in Figure 1, a second Dunnage Load Station 21 can be employed. In a preferred embodiment of this practice, second Dunnage Station 21 is allocated to a different type of dunnage than utilized in Station 19. For example, if 19 employs U-board, Station 21 can be allocated to peanuts or shredded paper; this lends flexibility to the packing system of the invention in circumstances where the product cartons and/or shipping containers are of different sizes and require mixing to fulfill a particular order.

### Solution Load:

In another optional practice, if a particular order requires an article, such as a container of saline or other solution, or indeed any article of manufacture affiliated with an order or a given product or an item otherwise desired to be included with the shipment, such can be packed into the shipping container as part of the instant invention. For example, if a particular order requires saline solution as part of the customer order, accessed as part of the order information available through the one or more databases available to the one or more computers associated with and controlling the packing operation of the present invention, it is transferred into the shipping container, using the label identifier thereon to track the shipping container through the packing system of the invention and enabling cross reference of same with the particular order having the request for solution, at, e.g. Solution Load Station 20. Transfer of solution or other article in this regard can be achieved by means known in the art, e.g. by use of one or more grippers having a vacuum actuated placement head or the like, which obtains the one or more solution containers or other articles from the hopper and deposits it into the shipping container. Placement of solution and the like into the appropriate shipping container can be checked by means known in the art, including e.g. by monitoring the vacuum on the placement head. As indicated above, in instances where solution is added to the shipping container, it is preferred that dunnage be dispensed with, the solution container itself providing the dunnage function.

At that point in the packing operation of the present invention when the entirety of a particular order has been packed into the requisite shipping container or containers, the contents can be checked to ensure accuracy and completeness before the container is sealed. In a preferred embodiment, this check is performed automatically through the databases; for example, using the label identifier on the shipping container, the position of same is tracked locally through the packing system; this coupled with the ability to confirm through the databases that the various steps, such as verifying placement of the invoice, products, dunnage, solution placement and the like, have successfully been implemented at the respective stations assigned to these tasks enables identification of improperly packed containers. Hence, if for example the proper number of product cartons has not been loaded, but the shipping container proceeds to the next station irrespectively, this will be tracked and the shipping container denominated a reject, e.g. will be denominated a partial order. Variations of this tracking protocol to identify rejected shipping containers will be apparent to those of skill in the art. Manual inspection of shipping container contents can also be performed to ensure the integrity of a particular order. In the preferred embodiment of the invention as depicted in Figure 1, one or more reject lanes are provided, only one, reject lane 22, being shown. As illustrated, it is preferred that this lane be located coextensive with or after the final packing step, the last step here as shown in Figure 1 being the optional second Dunnage Loading Station 21. Other reject lanes may be employed throughout the system, e.g. coextensive with or after each packing station, as desired. Defects causing a given shipping container to be rejected and e.g. routed onto conveyor reject lane 22, can be rectified, e.g. by supplying the items missing from the order, with the shipping container being reintroduced into the packing system for sealing and shipping, or being manually shipped thereafter.

### Sealing and Shipping:

In the preferred embodiment depicted at Figure 1, shipping containers that have all components required to fulfill a particular order proceed to the Sealing Station 23. In a preferred practice, where RSC shipping containers are employed, Sealing Station 23 comprises a top taper whereby, automatically, the upper flaps of the RSC are folded down and then taped. Preferably, the top of the shipping container is compressed slightly as the tape is applied to ensure integrity of the seal. A preferred top taper in this regard is the TBS 100 FC Side Belt Drive Pressure Sensitive Taper commercially available from Combi Packaging Systems. Other types and methods of sealing known to the artisan are also contemplated by the present invention. Once taped, the shipping container proceeds to out feed conveyors for delivery to the appropriate carrier of shipping. In the preferred practice shown at Figure 1, shipping containers having special carrier designations, such as U. S. Postal Service (USPS) Priority Mail and the like, proceed to out-feed conveyor 26, prior to which in a preferred embodiment, they are identified with appropriate tape and the like for that carrier at Special Carrier Tape Station 25 to signify, e.g. it is a USPS Priority Mail package; in one embodiment, three sides of the shipping container are so taped. As recognized by those in the art, any number of different special carrier out-feed conveyors and associated taping stations can be employed and are contemplated by the present invention. In the practice of the invention, the order information contained in the one or more databases, which order information also has the special carrier instructions for a particular order, is accessed by the computer or computers controlling the packing operation, which instructions are implemented as the shipping container for that particular order is tracked to the Sealing Station 23 using its label identifier and one or more scanners (not shown) at that Station. Thus for example, the host computer having access to the order information will direct the local controller or PLC to route a given shipping container Special Carrier Tape Station 25 and ultimately to out-feed conveyor 26 for delivery to that carrier. In the preferred embodiment shown in Figure 1, if no special carrier instructions accompany a particular order, the shipping container for same can exit the system by out-feed conveyor 24 to a default carrier or designation such as the U.S. Postal Service, non-priority mail.

## Claims

1. A dunnage article for a container having a crosswise dimension and upwardly directed sidewalls, said dunnage article comprising a piece of unitary sheet material of elongated shape, said sheet material having a central portion and at least two opposing end flaps that are delineated from said central portion by first transverse fold lines, said end flaps being proportioned to be bent along said first transverse fold lines and be seated against the inside of said upwardly directed sidewalls with a pressed fit when said dunnage article is placed within said container, said central portion having a length substantially equal to but less than the crosswise dimension of said container, said central portion having at least one opening therethrough sufficient to enable viewing of product located directly underneath said dunnage article after said dunnage article is placed within said container.

2. The dunnage article of Claim 1 wherein said central portion has an alignment notch thereon.

3. The dunnage article of Claim 1 wherein said alignment notch is offset.

4. The dunnage article of Claim 2 wherein said opposing end flaps each have a mid longitudinal slit therethrough.

5. The dunnage article of Claim 4 wherein said opposing end flaps have second transverse fold lines substantially parallel to said first transverse fold lines.

6. A dunnage article for a container into which is packed at least one layer of ophthalmic lens product cartons having identifier information thereon, said container having a length and a width and substantially vertical walls, said dunnage article comprising a unitary sheet material of generally rectangular shape, said sheet material having a central portion and at least two opposing end flaps each having an outer edge, each opposing end flap being delineated from said central portion by a first transverse fold line, said two opposing end flaps each being approximately one half the length of said central portion and each having a second transverse fold line located between said outer edge and said first transverse fold line, each of said opposing end flaps having a slit extending from said outer edge to said first transverse fold line; said central portion having a length substantially equal to but less that the length of said container, said central portion having an alignment notch and at least one through opening sufficient to enable viewing of said identifier information on said ophthalmic lens product cartons located directly underneath said dunnage article.
